Europäisches Patentamt

European Patent Office

Office européen des brevets

(19)

(11) EP 0 892 618 B1

(12) FASCICULE DE BREVET EUROPEEN

(45) Date de publication et mention
de la délivrance du brevet:
**19.02.2003 Bulletin 2003/08**

(51) Int Cl.⁷: **A61B 5/00**

(86) Numéro de dépôt international:
**PCT/IB97/00363**

(21) Numéro de dépôt: **97908461.3**

(22) Date de dépôt: **08.04.1997**

(87) Numéro de publication internationale:
**WO 97/037586 (16.10.1997 Gazette 1997/44)**

(54) **DISPOSITIF DE DETERMINATION DE LA PROFONDEUR D'ANESTHESIE**

VORRICHTUNG ZUR BESTIMMUNG DER NARKOSE-TIEFE

DEVICE FOR DETERMINING THE DEPTH OF ANAESTHESIA

(84) Etats contractants désignés:
**BE CH DE DK ES FR GB IT LI LU NL PT SE**

(30) Priorité: **09.04.1996 FR 9604547**

(43) Date de publication de la demande:
**27.01.1999 Bulletin 1999/04**

(73) Titulaire: **MCSA Medical Control S.A.**
**1228 Plan-Les-Ouates (CH)**

(72) Inventeur: **Cohen-Laroque, Emmanuel-S.**
**74160 Archamps (FR)**

(74) Mandataire: **Nithardt, Roland**
**Cabinet Roland Nithardt,**
**Conseils en Propriété Industrielle S.A.,**
**Y-Parc Scientifique et Technologique,**
**Chemin de la Sallaz**
**1400 Yverdon-les-Bains (CH)**

(56) Documents cités:
**EP-A- 0 232 234          WO-A-92/06632**
**DE-B- 2 430 788          US-A- 3 946 725**

# Description

La présente invention concerne un dispositif de détermination de la profondeur d'anesthésie d'un patient auquel au moins un produit anesthésiant est administré, comportant des moyens d'acquisition d'au moins un signal représentatif de l'activité du coeur du patient, des moyens de mise en forme de chaque signal, des moyens de détection d'ondes périodiques déterminées dans chaque signal mis en forme, des moyens pour mesurer les intervalles temporels entre lesdites ondes périodiques et des moyens d'affichage de la profondeur d'anesthésie

La profondeur d'anesthésie d'un patient est une grandeur qu'il est essentiel d'évaluer de façon aussi précise et objective que possible pour trouver les conditions optimales entre l'action analgésique et amnésique de l'agent anesthésique d'une part, et la minimisation du risque anesthésique d'autre part.

Les conditions optimales ne peuvent être atteintes que si la profondeur d'anesthésie peut être évaluée avec une précision et une objectivité suffisantes.

Il existe actuellement plusieurs procédés et dispositifs permettant de déterminer dans une certaine mesure, la profondeur d'anesthésie d un patient. Dans la plupart des cas, l'anesthésiste détermine cette profondeur d'anesthésie de manière empirique en fonction de signes extérieurs de souffrance du patient tels que la sudation frontale, les sécrétions lacrymales, le myosis oculaire ou en fonction d'une perturbation excessive du système cardio-vasculaire telle que tachycardie ou bradycardie, pression artérielle anormale, etc. Ces paramètres varient selon le patient et l'évaluation de la profondeur d'anesthésie dépend fortement de l'appréciation subjective de l'anesthésiste.

Un procédé pour déterminer la profondeur d'anesthésie est proposé dans la demande internationale de brevet publiée sous le N° WO 91/19453. Ce procédé comprend l'administration répétée d'une stimulation au patient, l'enregistrement de l'activité électrique du cerveau du patient après chaque stimulation, le traitement informatisé de ces enregistrements et la transformation des informations ainsi obtenues en indications concernant la profondeur d'anesthésie. Ce procédé implique l'administration d'une stimulation au patient, ce qui peut être peu pratique à réaliser. D'autre part, cette réponse est indépendante du système nerveux autonome et ne tient pas compte de l'état instantané du système nerveux autonome du patient.

Un autre procédé ainsi qu'un appareil destiné à fournir une mesure de la profondeur d'anesthésie d'un patient sont décrits dans la demande de brevet international publiée sous le N° WO 92/06632. Dans ce procédé, une série d'ondes R est analysée de façon statistique afin d'obtenir un relevé de la modification de la variation de la fréquence cardiaque provoquée par la respiration. La fréquence instantanée est comparée à la fréquence moyenne de façon à fournir une quantification de la profondeur d'anesthésie.

En pratique, ce procédé est généralement inutilisable dans la mesure où le patient est ventilé artificiellement durant l'intervention. Dans cette situation, le contrôle physiologique de la ventilation n'a plus d'influence sur la mécanique ventilatoire.

La publication européenne EP 0 232 234 concerne un dispositif pour déterminer la profondeur d'anesthésie basée sur un signal électrique généré à partir d'un paramètre optique influencé uniquement par la pression sanguine du patient, ce signal étant représentatif du rapport de la variation de ce paramètre avec la variation de la fréquence cardiaque de ce patient.

La présente invention se propose de fournir un dispositif d'usage relativement simple basé sur un procédé permettant de déterminer en temps réel et de façon individuelle pour chaque patient, la profondeur d'anesthésie selon la réactivité du système nerveux autonome sans faire intervenir de critère subjectif, en mesurant un ou plusieurs paramètres différents liés au coeur du patient.

Le but fixé par la présente invention est atteint par un dispositif tel que défini en préambule et caractérisé en ce qu'il comporte en outre des moyens pour calculer la position temporelle desdites ondes périodiques, des moyens pour former des séries numériques contenant un nombre donné d'intervalles temporels, des moyens de calcul d'une dimension fractale desdites séries numériques et des moyens de calcul d'un coefficient de normalisation.

Selon une forme de réalisation préférée du dispositif, les moyens de calcul d'une dimension fractale des séries numériques sont agencés pour calculer une dimension de corrélation desdites séries et les moyens de calcul du coefficient de normalisation sont agencés pour déterminer ledit coefficient en fonction de la valeur de la dimension de corrélation des séries, le produit dudit coefficient et de la dimension de corrélation d'au moins une série déterminant la profondeur d'anesthésie.

De façon avantageuse, les moyens d'acquisition d'au moins un signal représentatif de l'activité du coeur du patient sont agencés pour mesurer au moins un paramètre lié au coeur dudit patient tel qu'un électrocardiogramme, le débit sanguin, l'absorption lumineuse par le sang, la pression artérielle, la teneur en oxygène du sang ou un signal acoustique émis par le coeur du patient.

Les moyens de mise en forme peuvent comporter un amplificateur opérationnel agencé pour maintenir la tension de chaque signal entre deux bornes déterminées et un convertisseur analogique/numérique agencé pour échantillonner le signal sortant de l'amplificateur à une fréquence d'échantillonnage donnée.

La fréquence d'échantillonnage est avantageusement supérieure à 500 Hz.

Dans la forme de réalisation préférée, les moyens de détection d'ondes périodiques comportent un microprocesseur agencé pour calculer le maximum d'une fonction de corrélation entre chaque signal représentatif de l'activité du coeur du patient et un modèle théorique de ladite onde périodique. déterminée.

Le dispositif selon la présente invention comporte de préférence des moyens de mémorisation agencés pour mémoriser les instants auxquels la fonction de corrélation atteint un maximum.

Selon une forme de réalisation avantageuse, le dispositif comporte un dispositif d'alarme agencé pour émettre un signal d'alarme lorsque la profondeur d'anesthésie dépasse une valeur de seuil déterminée.

Selon une forme de réalisation préférée, le dispositif comporte des moyens de réglage des paramètres de détermination de la profondeur d'anesthésie.

Ces moyens de réglage peuvent comporter un organe de réglage du nombre d'intervalles temporels dans chaque série numérique. Le nombre d'intervalles temporels de chaque série numérique est de préférence compris entre 20 et 80.

Les moyens de réglage comportent également un organe de réglage de la valeur de seuil au-delà de laquelle le signal d'alarme est émis.

Les moyens d'affichage de la profondeur d'anesthésie peuvent avantageusement comporter un écran d'affichage à cristaux liquides.

La présente invention et ses avantages seront mieux compris en référence à la description suivante d'un mode de réalisation préféré et aux dessins annexés dans lesquels:

- la figure 1 est un schéma-bloc du procédé sur lequel est basé le dispositif de la présente invention,

- la figure 2 est un modèle théorique de diagramme électrique cardiaque en fonction du temps,

- la figure 3 est un exemple de diagramme expérimental d'intervalles entre les ondes R d'un patient en fonction du temps.

- la figure 4 est un diagramme de la dimension de corrélation du diagramme de la figure 3,

- la figure 5a est une vue schématique de l'intérieur du dispositif selon la présente invention, et

- la figure 5b est une vue schématique de l'extérieur du dispositif de la figure 5a.

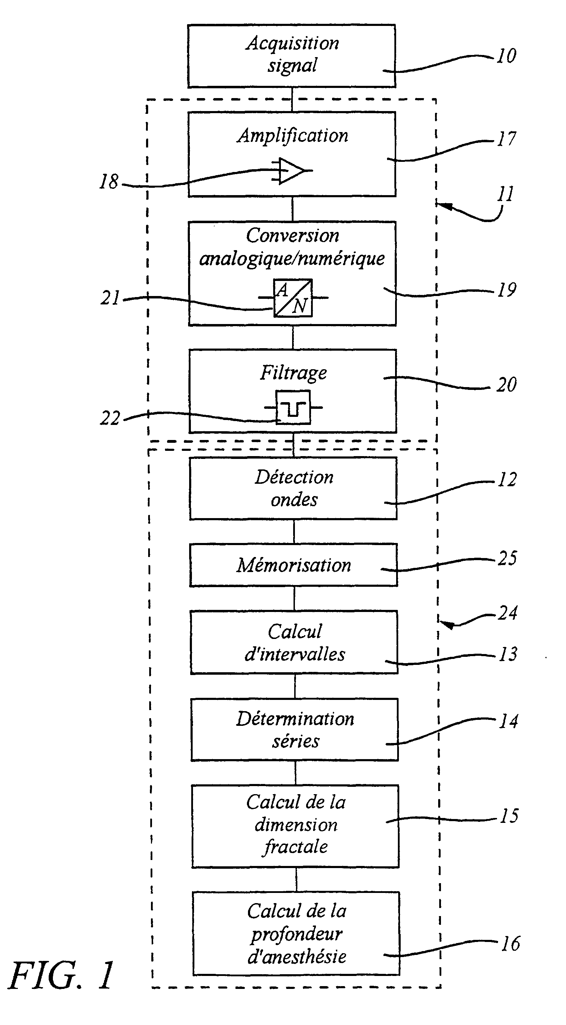

Le procédé sur lequel est basé le dispositif selon la présente invention est décrit ci-dessous en référence aux figures 1 à 4. Ce procédé comporte essentiellement une étape 10 d'acquisition d'un signal représentatif de l'activité du coeur d'un patient, une étape 11 de mise en forme de ce signal, une étape 12 de détection d'une onde périodique telle que par exemple une onde R dans le signal mis en forme, une étape 13 de calcul d'intervalles temporels entre lesdites ondes périodiques successives, une étape 14 de détermination de séries numériques d'intervalles temporels, une étape 15 de calcul d'une dimension fractale desdites séries d'intervalles temporels et une étape 16 de calcul de la profondeur d'anesthésie en fonction de la dimension fractale.

L'étape 10 d'acquisition du signal représentatif de l'activité du coeur du patient est réalisée de manière connue de l'homme du métier. Ce signal peut par exemple provenir d'un électrocardiographe. D'autres signaux peuvent être utilisés, et notamment un signal correspondant au débit sanguin du patient, à l'absorption lumineuse par son sang, sa pression artérielle, sa teneur en oxygène ou les ondes sonores émises par son coeur.

Les électrocardiographes couramment utilisés délivrent un signal électrique analogique dont la tension varie entre -1V et +1V, entre -5V et +5V ou entre -10V et +10V. La mise en forme 11 de ce signal comporte une étape 17 de normalisation consistant à l'amplifier au moyen d'un amplificateur opérationnel 18 de façon que le signal résultant varie par exemple entre -5V et +5V quel que soit le type d'électrocardiographe utilisé. Le gain de cet amplificateur opérationnel peut être modifié automatiquement en fonction de l'amplitude du signal d'entrée. Cette mise en forme comporte également une étape 19 de conversion de ce signal analogique en un signal numérique, et une étape 20 de filtrage de ce signal numérique.

L'étape de conversion 19 consiste à introduire le signal de sortie de l'amplificateur 18 dans un convertisseur analogique/numérique 21 qui échantillonne le signal à une fréquence d'échantillonnage supérieure à 500 Hz et, par exemple, à une fréquence comprise entre 500 Hz et 2kHz. La résolution de l'échantillonnage peut être supérieure à 8 bits et par exemple être égale à 13 bits. L'étape de filtrage 20 consiste à introduire le signal dans un filtre 22 conventionnel de manière à éliminer une composante ayant une fréquence correspondant à la fréquence du secteur utilisé pour l'alimentation électrique.

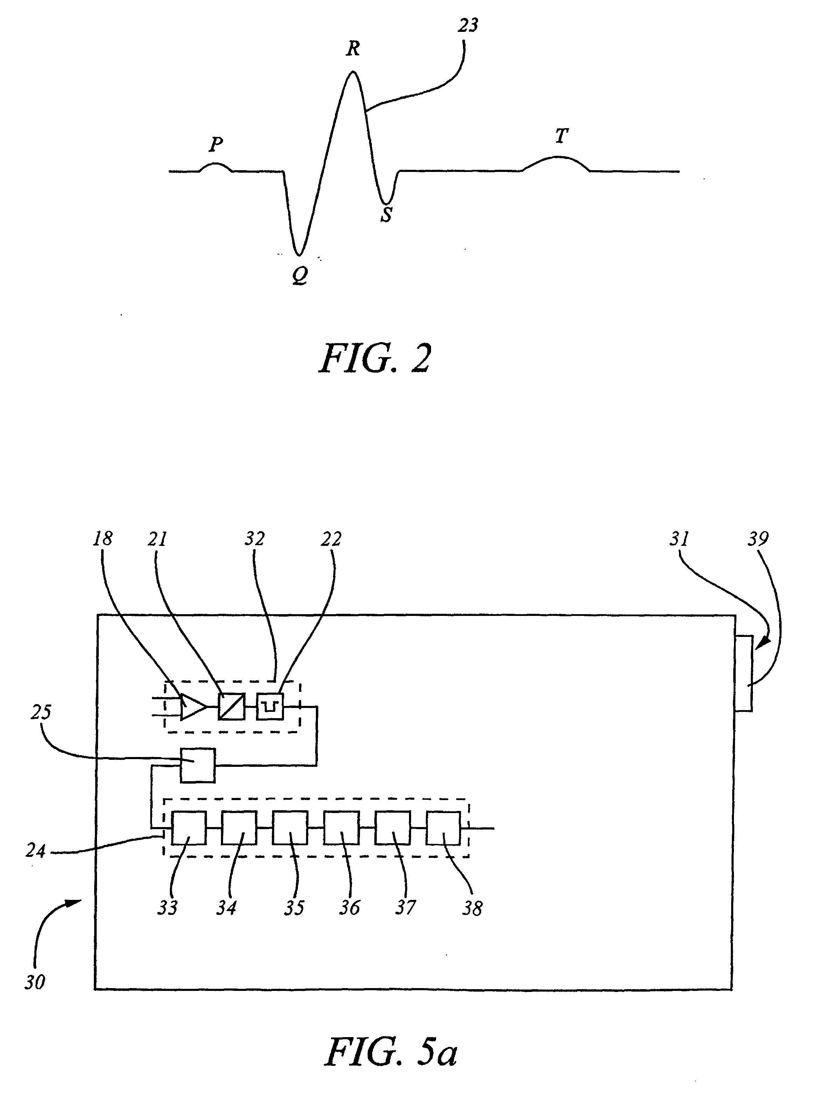

La figure 2 illustre un modèle théorique de signal sortant d'un électrocardiographe. Ce signal comporte notamment une zone ayant une forte amplitude positive, connue de l'homme du métier sous le nom d'onde R et portant la référence 23 dans la figure 2. L'étape 12 consiste à détecter la position dans le temps de ces ondes R 23. Cette détection peut se faire en utilisant un procédé connu sous le nom de "digital matched filtering" consistant à calculer le maximum d'une fonction de corrélation de deux signaux. L'un des signaux est le signal échantillonné sortant du filtre 22 et l'autre signal est un signal théorique représentant l'onde R. Ce procédé consiste à décaler l'onde R "théorique" par rapport au signal réel et à mesurer la corrélation en fonction du décalage. Cette corrélation s'exprime mathématiquement par:

$$\varphi_{xy}(k) = \sum_{l=0}^{N-1} x(l-k)y(l)$$

où $x$ est le signal réel sortant du filtre, $y$ est le signal théorique, $l$ est l'indice correspondant au numéro d'échantillon du signal échantillonné et $N$ est le nombre d'échantillons du signal échantillonné.

Ce calcul est réalisé au moyen d'un microprocesseur 24 qui peut être par exemple un microprocesseur commercialisé par Texas Instrument sous le nom de Digital Signal Processing TMS 320 C25.

La valeur de I correspondant au maximum de la fonction de corrélation $\varphi_{xy}$ indique la position temporelle de l'onde R. Cette valeur est mémorisée dans des moyens de mémorisation 25 associés par exemple au microprocesseur 24. Ce calcul est répété de façon à détecter la position de toutes les ondes R en fonction du temps.

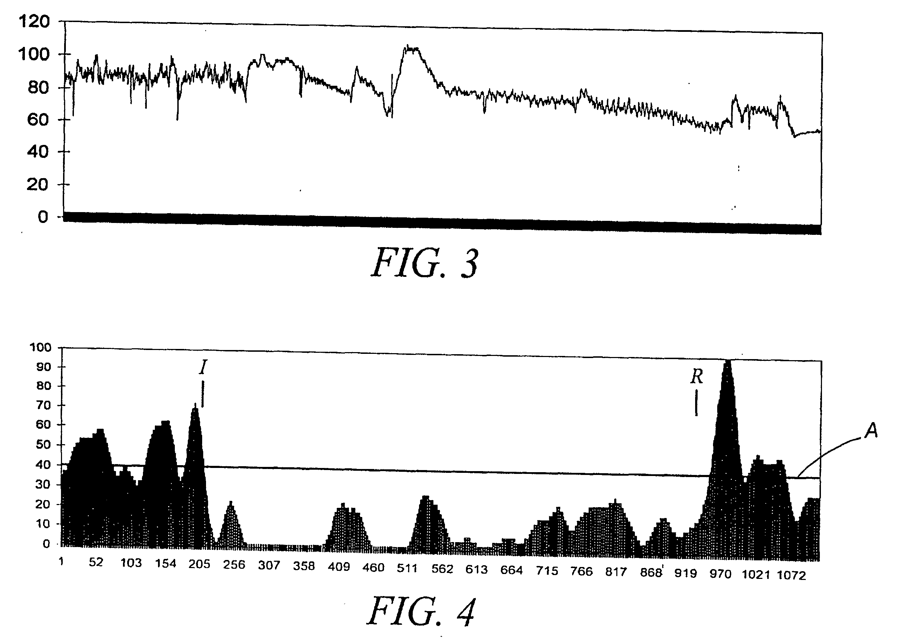

L'étape 13 suivante consiste à calculer l'intervalle temporel qui sépare deux ondes R successives. Tous ces intervalles calculés sont mémorisés dans les moyens de mémorisation 25. Le graphe illustré par la figure 3 représente les intervalles R-R en fonction du temps.

Dans l'étape 14, ces intervalles sont ensuite regroupés pour former des séries numériques comportant un certain nombre d'intervalles R-R. De manière concrète, plus le nombre d'intervalles R-R utilisés pour constituer une série numérique est grand, plus la précision et la fiabilité de la mesure seront grandes. Toutefois, plus ce nombre d'intervalles est grand, plus le temps d'attente entre une modification de la profondeur d'anesthésie d'un patient et l'affichage de cette modification par l'appareil sera long. Il est donc nécessaire de trouver un compromis entre ces deux contraintes. En pratique, une série comportant entre 20 et 80 intervalles et de préférence environ 40 intervalles donne des résultats fiables et rapides.

La phase suivante consiste à déterminer la profondeur d'anesthésie en fonction des informations concernant les séries numériques d'intervalles R-R.

Les pulsations cardiaques sont générées dans le coeur et sont régulées par un certain nombre de paramètres associés notamment au système nerveux autonome. Le nombre de paramètres est directement lié à la dimension fractale d'une série numérique d'intervalles R-R. L'administration d'un ou de plusieurs anesthésiants à un patient modifie le nombre de paramètres qui régulent sa fonction cardiaque et par conséquent la dimension fractale de la série d'intervalles R-R correspondant à sa pulsation cardiaque. Le calcul de la dimension fractale donne donc une mesure de la profondeur d'anesthésie.

L'étape 15 consiste à calculer la dimension fractale de chaque série numérique d'intervalles R-R. Cette dimension fractale peut être approximée en déterminant une dimension de corrélation des séries numériques. Cette dimension de corrélation peut se calculer en utilisant la formule suivante:

$$D = \lim_{r \to 0} \frac{\log C(r)}{\log r}$$

où $C(r)$ est la somme de corrélation.

L'étape 16 consiste à transformer cette dimension de corrélation en données représentant la profondeur d'anesthésie. Afin d'adapter le procédé à chaque patient individuel, la dimension de corrélation est déterminée avant l'injection de l'anesthésiant. La moyenne entre plusieurs valeurs de la dimension de corrélation obtenues pour un patient donné avant l'injection est multipliée par un coefficient de normalisation de telle façon que le produit corresponde environ à une valeur arbitraire donnée, par exemple de 100 unités.

La profondeur d'anesthésie est alors égale à la dimension de corrélation d'une série numérique d'intervalles R-R ou d'une moyenne de dimensions de corrélation, multipliée par le coefficient de normalisation défini ci-dessus. En pratique, il peut être avantageux de calculer une moyenne de dimensions de corrélation ou dé profondeurs d'anesthésie afin d'obtenir une valeur plus stable. Généralement, la moyenne est calculée sur un nombre de séries compris entre deux et dix. Il est toutefois également possible de n'utiliser qu'une série pour chaque calcul. Il est également possible d'utiliser une variation ou une corrélation de plusieurs séries

La figure 4 illustre sous forme d'histogrammes, la profondeur d'anesthésie d'un patient, déduite du graphe de la figure 3. La partie de cette figure située à gauche de la ligne verticale identifiée par I représente la profondeur d'anesthésie avant l'injection du produit anesthésiant. La ligne I représente l'injection du produit anesthésiant. La ligne verticale identifiée par R représente le réveil du patient et la zone comprise entre ces lignes I et R représente la période pendant laquelle le produit anesthésiant agit. La ligne horizontale A représente une valeur de seuil que la profondeur d'anesthésie ne doit pas dépasser pendant l'anesthésie, c'est-à-dire entre I et R.

Certains paramètres du procédé peuvent être modifiés de façon à adapter la réponse et la sensibilité de l'appareil au patient traité et aux opérations effectuées. Ces paramètres sont notamment la taille de la série temporelle (nombre d'intervalles R-R) utilisée lors du calcul de la dimension de corrélation et le nombre de dimensions de corrélation utilisées pour calculer la moyenne de la profondeur d'anesthésie. Il est en outre possible d'effectuer d'autres calculs statistiques tels que fluctuations, déviation standard, écart-type, etc.,

Il est également possible de fixer la valeur de seuil de la profondeur d'anesthésie correspondant à la ligne horizontale A de la figure 4. Si la profondeur d'anesthésie dépasse cette valeur, une alarme est déclenchée de façon à indiquer à l'anesthésiste que le patient est en train de se réveiller. Il peut donc être nécessaire de le réanesthésier. Sur la figure 4, cette valeur de seuil est fixée à 40 unités. Elle est déterminée par l'anesthésiste pour chaque patient.

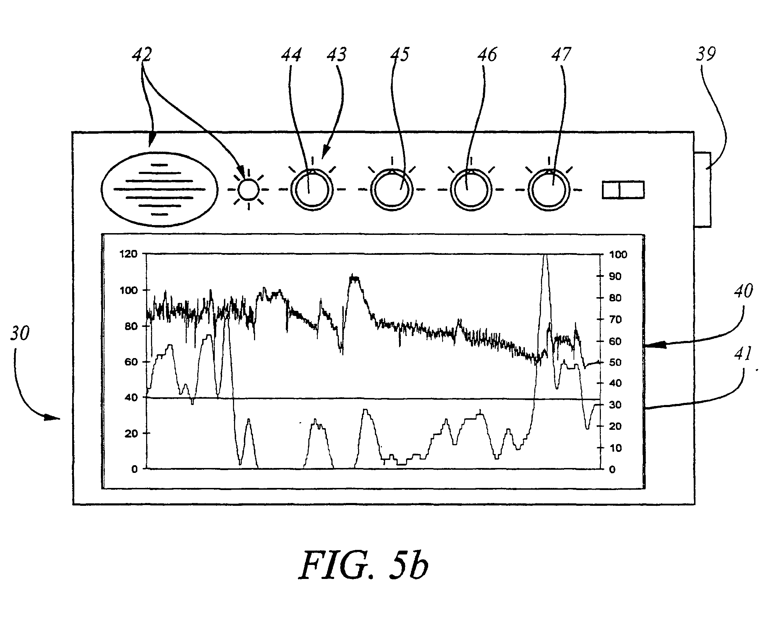

Le dispositif 30 selon la présente invention, réalisé sur la base du procédé décrit, est illustré par les figures 5a et 5b. Ce dispositif comporte essentiellement des moyens 31 d'acquisition d'au moins un signal représentatif de l'activité du coeur du patient, des moyens 32 de mise en forme de chaque signal, des moyens 33 de détection d'ondes périodiques, des moyens 34 pour calculer la position temporelle desdites ondes périodiques, des moyens 35 pour mesurer les intervalles, des moyens 36 pour former des séries numériques, des moyens 37 de calcul d'une dimension fractale et des moyens 38 de calcul d'un coefficient de normalisation. Les moyens 33 à 38 ainsi que les moyens de mémorisation 25 peuvent tous être formés par le microprocesseur 24.

Les moyens d'acquisition 31 comportent une prise de connexion 39 permettant de connecter le dispositif 30 à la sortie d'un électrocardiographe par exemple. Les moyens 32 de mise en forme du signal sont formés de l'amplificateur opérationnel 18, du convertisseur analogique/numérique 21 et du filtre 22. L'amplificateur permet de normaliser l'amplitude maximale du signal de sortie de l'électrocardiographe, afin que cette amplitude varie toujours entre deux mêmes bornes quelle que soit l'amplitude de sortie maximale de l'électrocardiographe. Le convertisseur analogique/numérique permet d'échantillonner le signal sortant de l'amplificateur et le filtre permet d'éliminer des fréquences parasites et notamment des fréquences dues à l'alimentation par secteur.

Le dispositif 30 comporte également des moyens d'affichage 40 de la profondeur d'anesthésie constitués par exemple d'un écran d'affichage 41 à cristaux liquides. Cet écran d'affichage permet de visualiser différentes informations de façon à les rendre immédiatement accessibles. L'information qui est toujours affichée à l'écran est la dimension de corrélation. Cette information est par exemple affichée sous forme d'histogrammes en fonction du temps, comme cela est représenté par les figures 4 et 5b. Une autre information utile est la valeur de chaque intervalle R-R en fonction du temps. Une modification de la dimension de corrélation peut provenir d'un changement d'état d'éveil du patient, auquel cas il est nécessaire de réagir en administrant un anesthésiant supplémentaire, mais peut également provenir d'une déstabilisation du patient due par exemple à une obstruction pulmonaire ou à une hémorragie. Dans ce cas, la réaction à avoir n'est pas la même. L'affichage simultané des intervalles R-R et de la profondeur d'anesthésie permet d'évaluer l'état du patient et les modifications de cet état en un seul coup d'oeil. Il est également possible d'afficher une ligne représentant la profondeur d'anesthésie au-delà de laquelle un signal d'alarme doit se déclencher, de façon que l'anesthésiste puisse évaluer facilement l'écart entre la profondeur d'anesthésie instantanée et la profondeur critique, de manière à pouvoir anticiper les modifications de l'état du patient.

Le dispositif 30 comporte également un dispositif d'alarme 42 agencé pour déclencher une alarme sonore et/ou visuelle lorsque la profondeur d'anesthésie du patient dépasse une valeur de seuil prédéfinie.

Finalement, le dispositif 30 comporte des moyens 43 de réglage des paramètres nécessaires à la détermination de la profondeur d'anesthésie. Ces moyens comportent notamment un organe de réglage 44 permettant de sélectionner le nombre d'intervalles R-R par série, un organe de réglage 45 permettant de choisir le nombre de séries d'intervalles R-R utilisées pour chaque affichage de la dimension de corrélation. En pratique, plus le nombre de séries est grand, plus la valeur est stable, mais plus les modifications de l'état du patient sont détectées tardivement. Le nombre de séries utilisées est généralement compris entre I et 10.

Les moyens de réglage 43 comportent également un organe de réglage 46 du coefficient de normalisation entre la dimension de corrélation et la profondeur d'anesthésie. Ce coefficient de normalisation peut par exemple être réglé soit manuellement par le réglage d'un potentiomètre, soit automatiquement par le dispositif 30.

Les moyens de réglage 43 comportent également un organe de réglage 47 du niveau d'alarme permettant de régler la valeur de la profondeur d'anesthésie au-delà de laquelle l'alarme doit se déclencher. Ce réglage est fait par l'anesthésiste en fonction des risques présentés par le patient et de la phase de l'opération qu'il subit.

Le dispositif selon la présente invention peut être alimenté par le secteur, dans le cas où il est utilisé en salle d'opération, et par batterie dans le cas où il est déplacé par exemple depuis la salle d'opération jusqu'à une salle de réveil.

Ce dispositif est particulièrement intéressant par le fait qu'il permet une évaluation objective de la profondeur d'anesthésie. Ceci permet un dosage plus précis de l'anesthésiant ce qui entraîne à la fois une optimisation du coût de l'agent pharmacologique et une diminution du risque anesthésique. L'évaluation précise de la profondeur d'anesthésie permet également de tester de façon plus fiable et objective de nouveaux agents anesthésiques. Il permet en outre une analyse objective et précise de la fonction du système nerveux autonome des patients.

Le dispositif selon la présente invention n'étant pas directement connecté au patient, les normes de protection médicale sont plus souples et sa réalisation en est fortement simplifiée. Le faible encombrement de ce dispositif permet en outre son installation rapide sur des tours de monitorage standards.

Ce dispositif permet à l'anesthésiste de réagir rapidement à une modification de la profondeur d'anesthésie d'un patient, avant de voir apparaître les premiers signes de douleur. Comme on constate qu'un meilleur vécu de l'anesthésie permet une meilleure récupération, cet appareil permet en outre de diminuer les problèmes postopératoires.

La présente invention n'est pas limitée au mode de réalisation décrit. Il est possible d'utiliser d'autres signaux que le signal de sortie d'un électrocardiographe. En particulier, dans le cas d'une opération nécessitant l'ouverture du thorax du patient, l'électrocardiogramme ne peut pas être mesuré. Dans ce cas, on peut utiliser la pression artérielle du patient, son débit sanguin mesuré par exemple par effet Doppler ou tout autre paramètre lié, tel que par exemple, l'absorption lumineuse du patient, la teneur en oxygène de son sang ou des signaux acoustiques émis par son coeur. Il est également possible de mesurer plusieurs de ces paramètres, de calculer une profondeur d'anesthésie pour chacun d'eux et de combiner ces profondeurs d'anesthésie. Ceci permet d'obtenir un résultat plus fiable et une plus grande sécurité lors de l'anesthésie.

## Revendications

1. Dispositif de détermination de la profondeur d'anesthésie d'un patient auquel au moins un produit anesthésiant est administré comportant des moyens (31) d'acquisition d'au moins un signal représentatif de l'activité du coeur du patient, des moyens (32) de mise en forme de chaque signal, des moyens (33) de détection d'ondes périodiques déterminées dans chaque signal mis en forme, des moyens (35) pour mesurer les intervalles temporels entre lesdites ondes périodiques et des moyens (40) d'affichage de la profondeur d'anesthésie, **caractérisé en ce qu'**il comporte en outre des moyens (34) pour calculer la position temporelle desdites ondes périodiques, des moyens (36) pour former des séries numériques contenant un nombre donné d'intervalles temporels, des moyens (37) de calcul d'une dimension fractale desdites séries numériques et des moyens (38) de calcul d'un coefficient de normalisation.

2. Dispositif selon la revendication 1, **caractérisé en ce que** les moyens (37) de calcul d'une dimension fractale des séries numériques sont agencés pour calculer une dimension de corrélation desdites séries.

3. Dispositif selon les revendications 1 et 2, **caractérisé en ce que** les moyens (38) de calcul du coefficient de normalisation sont agencés pour déterminer ledit coefficient en fonction de la valeur de la dimension de corrélation des séries, le produit dudit coefficient et de la dimension de corrélation d'au moins une série déterminant la profondeur d'anesthésie.

4. Dispositif selon la revendication 1, **caractérisé en ce que** les moyens (31) d'acquisition d'au moins un signal représentatif de l'activité du coeur du patient sont agencés pour mesurer au moins un paramètre lié au coeur dudit patient, tel qu'un électrocardiogramme, le débit sanguin, l'absorption lumineuse par le sang, la pression artérielle, la teneur en oxygène du sang ou un signal acoustique émis par le coeur du patient.

5. Dispositif selon la revendication 1, **caractérisé en ce que** les moyens (32) de mise en forme comportent un amplificateur opérationnel (18) agencé pour maintenir la tension de chaque signal entre deux bornes déterminées et un convertisseur analogique/numérique (21) agencé pour échantillonner le signal sortant de l'amplificateur à une fréquence d'échantillonnage donnée.

6. Dispositif selon la revendication 5, **caractérisé en ce que** la fréquence d'échantillonnage est supérieure à 500 Hz.

7. Dispositif selon la revendication 1, **caractérisé en ce que** les moyens (33) de détection d'ondes périodiques comportent un microprocesseur (24) agencé pour calculer le maximum d'une fonction de corrélation entre le signal représentatif de l'activité du coeur du patient et un modèle théorique de ladite onde périodique déterminée.

8. Dispositif selon la revendication 7, **caractérisé en ce qu'**il comporte des moyens de mémorisation (25) agencés pour mémoriser les instants auxquels la fonction de corrélation atteint un maximum.

9. Dispositif selon la revendication 1, **caractérisé en ce qu'**il comporte un dispositif d'alarme (42) agencé pour émettre un signal d'alarme lorsque la profondeur d'anesthésie dépasse une valeur de seuil déterminée.

10. Dispositif selon la revendication 1, **caractérisé en ce qu'**il comporte des moyens de réglage (43) des paramètres de mesure.

11. Dispositif selon la revendication 10, **caractérisé en ce que** les moyens de réglage (43) comportent un organe de réglage (44) du nombre d'intervalles temporels dans chaque série numérique.

12. Dispositif selon la revendication 11, **caractérisé en**

**ce que** le nombre d'intervalles temporels de chaque série numérique est compris entre 20 et 80.

13. Dispositif selon les revendications 10 et 11, **caractérisé en ce que** les moyens de réglage (43) comportent un organe de réglage (47) de la valeur de seuil (A) au-delà de laquelle le signal d'alarme est émis.

14. Dispositif selon la revendication 1, **caractérisé en ce que** les moyens d'affichage (40) de la profondeur d'anesthésie comportent un écran d'affichage (41) à cristaux liquides.

**Claims**

1. A device for determining the depth of anaesthesia in a patient to whom at least one anaesthetic substance is administered, comprising means (31) for acquiring at least one signal representing the patient's cardiac activity, means (32) for configuring each signal, means (33) for detecting periodic waves determined for each configured signal, means (35) for measuring the time intervals between said periodic waves and means (40) for displaying the depth of anaesthesia, **characterized in that** it further comprises means (34) for calculating the temporal position of said periodic waves, means (36) for forming digital series containing a given number of time intervals, means (37) for calculating a fractal dimension of said digital series and means (38) for calculating a coefficient of normalisation.

2. A device according to claim 1, **characterized in that** means (37) for calculating a fractal dimension of the digital series is designed for calculating a dimension of correlation for said series.

3. A device according to claims 1 and 2, **characterized in that** means (38) for calculating a coefficient of normalisation is designed for determining said coefficient as a function of the value of the dimension of correlation for the series, the product of said coefficient and the dimension of correlation of at least one series determining the depth of anaesthesia.

4. A device according to claim 1, **characterized in that** means (31) for acquiring at least one signal representing the patient's cardiac activity is designed for measuring at least a parameter concerning said patient's heart such as electrocardiogram, blood flow velocity, light absorption factor of the blood, blood pressure, oxygen content of the blood or acoustic signal emitted by the patient's heart.

5. A device according to claim 1, **characterized in**

**that** the configuring means (32) comprises an operational amplifier (18) designed to maintain the voltage of each signal between two predetermined limits and an analog/digital converter (21) designed to sample the signal coming from the amplifier at a given sampling frequency.

6. A device according to claim 5, **characterized in that** the sampling frequency is higher than 500 Hz.

7. A device according to claim 1, **characterized in that** the means (33) for detecting periodic waves comprises a microprocessor (24) designed to calculate the maximum of a function of correlation between the signal representing cardiac activity of the patient and a theoretical model of said predetermined periodic wave.

8. A device according to claim 7, **characterized in that** it comprises storage means (25) designed to record the instants when the function of correlation attains a maximum.

9. A device according to claim 1, **characterized in that** it comprises an alarm device (42) designed to produce a signal when the depth of anaesthesia exceeds a predetermined threshold level.

10. A device according to claim 1, **characterized in that** it comprises means (43) for regulating measurement parameters.

11. A device according to claim 10, **characterized in that** the regulation means (43) comprises of a device (44) for regulating the number of time intervals in each digital series.

12. A device according to claim 11, **characterized in that** the number of time intervals in each digital series ranges from 20 to 80.

13. A device according to claims 10 and 11, **characterized in that** the regulation means (43) comprises a device (47) for regulating the threshold value (A) above which the alarm signal is emitted.

14. A device according to claim 1, **characterized in that** the means (40) for displaying the depth of anaesthesia comprises a liquid crystal display screen (41).

**Patentansprüche**

1. Vorrichtung zur Bestimmung der Narkotisierungstiefe eines Patienten, dem mindestens ein narkotisierendes Produkt verabreicht wird, das Mittel (31) zur Erfassung mindestens eines für die Herzaktivität des Patienten repräsentativen Signals, Mittel (32) zur Aufbereitung eines jeden Signals, Mittel (33) zur Erkennung von in jedem aufbereiteten Signal bestimmten, wiederkehrenden Wellen, Mittel (35) zum Messen der Zeitabstände zwischen den sogenannten regelmäßig wiederkehrenden Wellen und Mittel (40) zum Anzeigen der Narkotisierungstiefe aufweist,

**dadurch gekennzeichnet, dass**

sie außerdem Mittel (34) zur Berechnung der zeitlichen Position der regelmäßig wiederkehrenden Wellen, Mittel (36) zur Ausbildung von eine Anzahl gegebener Zeitabstände enthaltenden Zahlenreihen, Mittel (37) zur Berechnung einer Fraktalen der Zahlenreihen und Mittel (38) zur Berechnung eines Standardisierungskoeffizienten aufweist.

2. Vorrichtung nach Anspruch 1,
**dadurch gekennzeichnet, dass**
die Mittel (37) zur Berechnung einer Fraktalen von Zahlenreihen zur Berechnung einer Wechselbeziehungsgröße der Reihen vorgesehen sind.

3. Vorrichtung nach den Ansprüchen 1 und 2,
**dadurch gekennzeichnet, dass**
die Mittel (38) zur Berechnung des Standardisierungskoeffizienten zur Bestimmung des Koeffizienten in Abhängigkeit von dem Wert der Wechselbeziehungsgröße der Reihen, des Produktes des Koeffizienten und der Wechselbeziehungsgröße mindestens einer die Narkotisierungstiefe bestimmenden Reihe vorgesehen sind.

4. Vorrichtung nach Anspruch 1,
**dadurch gekennzeichnet, dass**
die Mittel (31) zur Erfassung mindestens eines für die Herzaktivität des Patienten repräsentativen Signals zum Messen mindestens eines mit dem Herz des Patienten in Zusammenhang stehenden Parameters, wie z. B. eines EKGs, des Blutdurchsatzes, der Lichtabsorption des Blutes, des arteriellen Druckes, des Sauerstoffgehaltes des Blutes oder eines von dem Herzen des Patienten ausgehenden akustischen Signals vorgesehen sind.

5. Vorrichtung nach Anspruch 1,
**dadurch gekennzeichnet, dass**
die Mittel (32) zur Aufbereitung einen Operationsverstärker (18) umfassen, der zur Aufrechterhaltung der Spannung von jedem Signal zwischen zwei festgelegten Klemmen und einem zur Entnahme von Mustern von dem aus dem Verstärker austretenden Signal mit einer gegebenen Musterentnahmefrequenz vorgesehenen Analog-Digital-Umsetzer (21) vorgesehen ist.

6. Vorrichtung nach Anspruch 5,
**dadurch gekennzeichnet, dass**
die Musterentnahmefrequenz größer als 500 Hz ist.

7. Vorrichtung nach Anspruch 1,
**dadurch gekennzeichnet, dass**
die Mittel (33) zur Erkennung von bestimmten, wiederkehrenden Wellen einen zur Berechnung des Maximums einer Wechselbeziehungsfunktion zwischen dem für die Herzaktivität des Patienten repräsentativen Signal und einem theoretischen Modell der bestimmten, regelmäßig wiederkehrenden Welle vorgesehenen Mikroprozessor (24) umfassen.

8. Vorrichtung nach Anspruch 7,
**dadurch gekennzeichnet, dass**
sie Speichermittel (25) umfasst, die zum Speichern der Zeitpunkte vorgesehen sind, an denen die Wechselbeziehungsfunktion ein Maximum erreicht.

9. Vorrichtung nach Anspruch 1,
**dadurch gekennzeichnet, dass**
sie eine Alarmvorrichtung (42) umfasst, die zur Ausgabe eines Alarmsignals vorgesehen ist, wenn die Narkotisierungstiefe einen festgelegten Schwellenwert unterschreitet.

10. Vorrichtung nach Anspruch 1,
**dadurch gekennzeichnet, dass**
sie Mittel (43) zum Einstellen der Messparameter umfasst.

11. Vorrichtung nach Anspruch 10,
**dadurch gekennzeichnet, dass**
die Mittel (43) zum Einstellen ein Element (44) zur Einstellung der Anzahl von Zeitabständen in jeder Zahlenreihe umfassen.

12. Vorrichtung nach Anspruch 11,
**dadurch gekennzeichnet, dass**
die Anzahl der Zeitabstände jeder Zahlenreihe zwischen 20 und 80 beträgt.

13. Vorrichtung nach den Ansprüchen 10 und 11,
**dadurch gekennzeichnet, dass**
die Mittel (43) zum Einstellen ein Element (47) zur Einstellung des Schwellenwertes (A) umfassen, ab welchem das Alarmsignal ausgegeben wird.

14. Vorrichtung nach Anspruch 1,
**dadurch gekennzeichnet, dass**
die Mittel (40) zum Anzeigen der Narkotisierungstiefe einen Flüssigkristall-Anzeigebildschirm (41) umfassen.

| Acquisition signal | — 10 |
| --- | --- |

| Amplification | — 17 |

18 —

| Conversion analogique/numérique | — 19 |

21 —

11

| Filtrage | — 20 |

22 —

| Détection ondes | — 12 |

| Mémorisation | — 25 |

24

| Calcul d'intervalles | — 13 |

| Détermination séries | — 14 |

| Calcul de la dimension fractale | — 15 |

| Calcul de la profondeur d'anesthésie | — 16 |

*FIG. 1*

FIG. 2

FIG. 5a

*FIG. 3*

*FIG. 4*

EP 0 892 618 B1

FIG. 5b